# EUROPEAN PATENT APPLICATION

(11) **EP 4 086 346 A1**
(43) Date of publication of application: **09.11.2022**
(21) Application number: 20909536.3
(22) Date of filing: 30.12.2020
(51) Int. Cl.: C12N 15/113, C12N 15/11, A61K 31/7115, A61K 48/00

(54) **METHOD FOR TREATING USHER SYNDROME AND COMPOSITION THEREOF**

(30) Priority: 30.12.2019 WO PCT/CN2019/129957
(71) Applicant: EdiGene Therapeutics (Beijing) Inc., Beijing 102206 (CN)
(72) Inventor: LIU, Nengyin, Beijing 102206 (CN); YI, Zexuan, Beijing 102206 (CN); YUAN, Pengfei, Beijing 102206 (CN); ZHAO, Yanxia, Beijing 102206 (CN); TANG, Gangbin, Beijing 102206 (CN)
(74) Representative: Lavoix
(86) International application number: PCT/CN2020/141501
(87) International publication number: WO 2021/136404

(57) **Abstract**

Provided is a method for targeted editing of target RNA containing a G to A mutation in a USH2A gene transcript based on LEAPER technology, comprising: introducing a construct of an adenosine deaminase recruiting RNA (arRNA) for editing the target RNA or a construct encoding said arRNA into a cell, wherein the arRNA comprises a complementary RNA sequence that hybridizes to the target RNA, and wherein the arRNA is capable of recruiting adenosine deaminase acting on RNA (ADAR), so that the target adenosine in the target RNA is deaminated, thereby performing in vivo editing of the base from A to I on RNA safely and effectively, repairing a pathogenic mutation site, and achieving the purpose of treating disease such as Usher syndrome.

## Description

### TECHNICAL FIELD

The present application belongs to the field of gene editing therapy, particularly relates to a method for targeted editing of gene mutations related to Usher syndrome type II based on LEAPER (leveraging endogenous ADAR for programmable editing on RNA) technology.

### BACKGROUND ART

Usher syndrome, also known as hereditary deafness-retinitis pigmentosa syndrome, was described and named by Charles Howard Usher in 1914¹². It is an autosomal recessive rare disease caused by gene mutation that causes congenital or progressive loss of vision and hearing. The incidence of Usher syndrome is approximately 1/12,500 in Germany⁸, 1/28,000 in Norway⁴, and 1/23,000 in the United States, and it is estimated that more than half of the 16,000 blind and deaf people in the United States have Usher syndrome¹. There are tens or even hundreds of thousands of patients worldwide who are in urgent need of treatment.

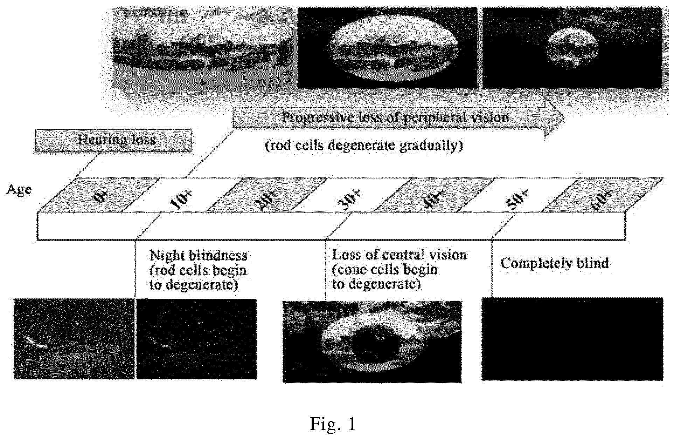

In 1977, Davenport et al. studied Usher syndrome and classified it into type I, II, III, and IV according to its severity³. Among them, type I is severe congenital deafness, a patient suffers from severe visual impairment around the age of 10; and type II is moderate to severe congenital deafness, a patient gradually loses vision between the ages of 10 and 50. Types III and IV are relatively rare, and the disease is mild; the auditory manifestations of a patient are progressive sensorineural deafness, and the time of visual loss is uncertain. Due to the early onset of type I, the intervention window period is short; while type III and IV are relatively mild. Vision loss in a type II patient gradually starts from 10 to 20 years old, suffering from night blindness at the beginning and eventually vision loss (Fig. 1), which allows us a longer therapeutic intervention window period and hopefully stop the gradual vision loss in a patient.

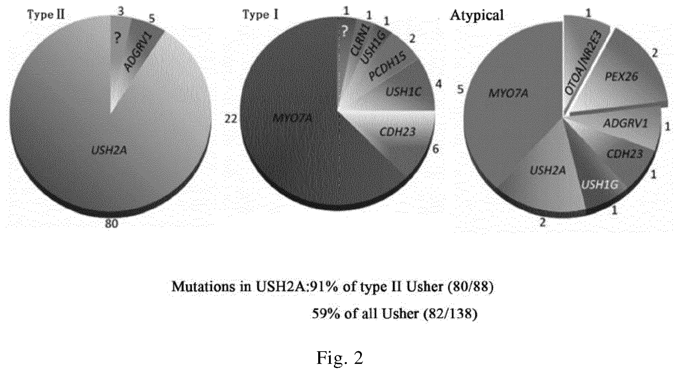

In 2017, Neuhaus et al. studied 138 patients with Usher syndrome by high-throughput sequencing. The results show that 82 of the 138 patients have USH2A gene mutations, and 80 of these 82 patients have Usher syndrome type II (Fig. 2)⁷. Therefore, when only Usher syndrome type II patients are considered, the proportion of USH2A gene mutations is more than 90% (Fig. 2)⁷, and the therapeutic regime for the USH2A gene is likely to benefit these patients.

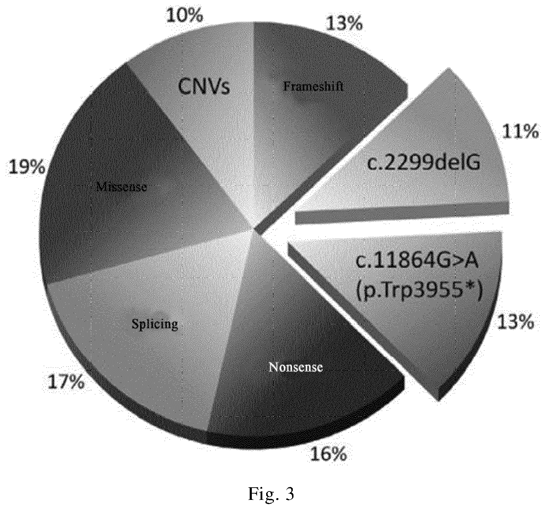

The USH2A gene encodes a protein called Usherin, which plays an important role in vision, and mainly exists in photosensitive cones and rods for connecting the inner segment and the outer segment. When USH2A is mutated, it causes the degenerative death of cone and rod cells⁵. A study by Neuhaus et al. in 2017 shows that, 13% of the USH2A gene mutation cases sequenced by them are NM_206933.2(USH2A)_c.11864G>A (p.Trp3955Ter), and this mutation type is a single mutation type having the largest proportion in all mutation types (Fig. 3)⁷.

Loss of the normal function of Usherin due to USH2A gene mutation is an important cause of USHER syndrome type II. Therapies targeting the USH2A gene become the main research and development direction of Usher type II treatment.

At present, there are two main research and development directions for the treatment of USHER syndrome type II through gene therapy. One is to mediate the complete USH2A gene by virus et al. to re-express it in the eyes. However, the protein sequence of USH2A gene is very long with a length of more than 6000 amino acids, which makes its corresponding coding region sequence having more than 18000 base pairs. The usual viral vectors have a limited delivery length. Typically, lentiviruses deliver no more than 10,000 base pairs, while adeno-associated viruses deliver no more than 4,500 base pairs. This makes it difficult to achieve the delivery of a full-length USH2A gene by viral vectors. Therefore, medical and scientific researchers can only choose to deliver the truncated USH2A gene. Although this method can alleviate the deterioration of the disease at a certain extent, it cannot fully achieve the original normal physiological function of Usherin due to the patient still lacks normal full-length Usherin.

Another common research and development direction for USH2A gene therapy is achieved through Exon Skipping. Since some USH2A gene mutations are caused by frame shift or nonsense mutation of a certain exon, as long as the exon can be specifically skipped in the process of RNA splicing, the sequence following the exon can be translated normally. It is common practice to introduce a short fragment of antisense nucleotides (anti-sense oligo, ASO) to specifically skip the exon targeted by the nucleotide to be translated during splicing. This method is similar to the previous method, but the full-length Usherin still cannot be obtained in the end due to skipping of the mutated exons.

In recent years, genome editing technology led by CRISPR (clustered regularly interspaced short palindromic repeats) is developing rapidly, and has a profound impact on many fields of biology and medicine. Many researchers and biotech companies are also working to bring this technology to clinic. In September 2019, an article published by Professor Deng Hongkui of Peking University and his collaborators reported the results of clinical trials on editing stem cells by CRISPR technology and infusing them back into a patient to treat their AIDS and leukemia, this made a huge contribution to the transformation of CRISPR technology in the field of gene therapy.

Although CRISPR technology has great application prospects, it also has a series of defects, which makes the transformation of this technology from the scientific research stage to clinical treatment application very difficult. One of the problems is the key enzyme used in CRISPR technology: Cas9. CRISPR-based DNA editing technology requires exogenous expression of Cas9 or other nucleases with similar function, which causes the following problems. Firstly, nucleases that require exogenous expression typically have large molecular weight, which drastically reduces the efficiency of their delivery into the body of a patient via viral vectors. Secondly, due to the exogenous expression of nucleases, this method has potential possibility of nuclease off-targets, which will lead to potential carcinogenic risks in its application. Finally, exogenously expressed Cas9 and other similar nucleases are found in bacteria, but not naturally occurring in humans or mammals, which makes it possible to elicit an immune response in patients, on one hand this may cause damage to the patients themselves, on the other hand the exogenously expressed nuclease may also be neutralized, thereby losing its due activity and affecting the therapeutic effect.

In 2017, Professor Zhang Feng from MIT and his research group reported an RNA editing technology called REPAIR (RNA editing for programmable A to I replacement), the editing of A into I in target RNA may be achieved through exogenously expressing of Cas13-ADAR fusion protein and single guide RNA (sgRNA), but same as CRISPR technology, this method still requires the expression of exogenous protein, and is still unable to solve the problem caused by foreign protein expression.

In January 2019, Thorsten Stafforst's group reported a single-base RNA editing technology called RESTORE (recruiting endogenous ADAR to specific trans for oligonucleotide-mediated RNA editing, Merkle et al., 2019). RESTORE can get rid of the dependence on foreign proteins, but the high editing efficiency of RESTORE technology needs the presence of IFN-γ, and IFN-γ is a key factor in determining the development and severity of autoimmunity, which makes the application of this technology in the medical field greatly reduced. On the other hand, a guide RNA is also used in the RESTORE technology, and the guide RNA used is a chemically synthesized oligonucleotide, and it needs to artificially introduce a large number of chemical modifications to the synthesized oligonucleotide to ensure its stability. Among these chemical modifications, some of them are non-natural modifications, which may make the oligonucleotide toxic or immunogenic; and some of the modifications will lead to different conformations of the same base chain, so that there may be dozens of different conformational combinations for the same RNA sequence, thereby increasing the difficulty of delivering the RNA into cells.

Since the above three techniques all have certain defects, and in view of the particularity of the USH2A gene itself, all of the above three techniques are difficult to be used for the treatment of USHER syndrome type II.

In July 2019, Professor Wei Wensheng's group from School of Life Sciences in Peking University published an article in Nature Biotechnology, "Programmable RNA editing by recruiting endogenous ADAR using engineered RNAs", which firstly reported a new nucleic acid editing technology: LEAPER (leveraging endogenous ADAR for programmable editing of RNA, Qu et al., 2019). On one hand, compared with CRISPR technology, this technology gets rid of the dependence on overexpression of exogenous nucleases in principle, and it only needs a fragment of RNA as a guide to recruit endogenous nucleases to the desired editing site, thus the technology has greater advantages in the process of transformation to the medical field; on the other hand, this technology only realizes the editing of adenosine A to creatinine I at the transcriptional level (creatinine I will be recognized as guanine G during protein translation), without changing the sequence in the genome, thus it is safer and more convenient. LEAPER technology can not only be accomplished by chemically synthesizing RNA, but also the RNA is delivered to patients through vectors such as adeno-associated virus (AAV) and lentivirus, thereby making the choice of delivery methods more flexible.

### SUMMARY OF THE INVENTION

The present application relates to a method for targeted editing of target RNA containing a G to A mutation in a USH2A gene transcript based on LEAPER technology, a construct comprising an adenosine deaminase recruiting RNA (arRNA) for editing target RNA or a construct encoding the arRNA is introduced into a cell containing the target RNA, so that the arRNA is able to recruit adenosine deaminase acting on the RNA (ADAR) thereby the target adenosine in the target RNA is deaminated. Through optimization and improvement of LEAPER technology, this application creatively applies it to target RNA containing a G to A mutation in a USH2A gene transcript, such as a target RNA related to USHER syndrome II with NM_206933.2 (USH2A)_c.11864G>A (p.Trp3955Ter) mutation, the purpose of treating USHER syndrome II is achieved through reversed repair of the mutation site.

The purpose of this application is to provide a new type of technical solution aiming at the pathogenic gene causing Usher syndrome type II, such as the pathogenic gene of mutation type NM_206933.2(USH2A)_c.11864G>A (p.Trp3955Ter) with the highest proportion in human USH2A, wherein precisely editing of the mutation site on the target RNA can be achieved without introducing oversize nucleic acid or exogenous protein molecule, thereby restoring the full function of Usherin protein.

Particularly, this application relates to:
1. A method for targeted editing of target RNA in a cell based on LEAPER technology, wherein the target RNA is an RNA containing a G to A mutation ins a USH2A gene (such as human USH2A gene) transcript, comprising:
   introducing a construct comprising an adenosine deaminase recruiting RNA (arRNA) for editing a target RNA or a construct encoding the arRNA into the cell, wherein the arRNA comprises a complementary RNA sequence that hybridizes to the target RNA, and wherein the arRNA is capable of recruiting adenosine deaminase acting on RNA (ADAR), thereby target adenosine in the target RNA is deaminated.
2. The method according to item 1, wherein the target RNA is a mature mRNA or an mRNA precursor (pre-mRNA). In some embodiments, the target RNA is an mRNA precursor (pre-mRNA).
3. The method according to item 1 or 2, wherein the length of the arRNA is about 151-61 nt, 131-66 nt, 121-66 nt, 111-66 nt, 91-66 nt, or 81-66 nt. This application covers any natural number within this numerical range.
4. The method according to item 3, wherein the length of the arRNA is any natural number selected from 66nt-131nt.
5. The method according to any one of items 1-4, wherein the length from the targeting base to the 3' end in the arRNA is ≥7nt, ≥10nt, ≥15nt, preferably 16-55nt, 20-50nt, 25-45nt, 25-35nt, or 25-30nt. This application covers any natural number within this numerical range.
6. The method according to any one of items 1-5, wherein the length from the targeting base to the 5' end in the arRNA is ≥25nt, ≥30, ≥35, ≥45, preferably 46-90nt, 50-85nt , 55-80nt, 60-75nt, or 65-70nt. This application covers any natural number within this numerical range.
7. The method according to any one of items 1-6, wherein a targeting base is introduced into the arRNA to pair with the target A in the target sequence, and the preference order of the targeting base from high to low is C, A, U, or G.
   In some embodiments, the targeting base introduced into the arRNA to pair with the target A in the target sequence is C. In some embodiments, the target base introduced into the arRNA to pair with the target A in the target sequence is A. In some embodiments, the targeting base introduced into the arRNA to pair with the target A in the target sequence is U.
8. The method according to any one of items 1-7, wherein the target RNA is a human USH2A gene RNA comprising a transcribed NM_206933.2(USH2A)_c.11864G>A (p.Trp3955Ter) mutation site.
9. The method according to any one of items 1-8, wherein the arRNA is selected from the group consisting of: SEQ ID NO: 23, SEQ ID NO: 25, SEQ ID NO: 26, SEQ ID NO: 27, SEQ ID NO: 28, SEQ ID NO: 29, SEQ ID NO: 30, SEQ ID NO: 17, SEQ ID NO: 18, SEQ ID NO: 19, SEQ ID NO: 11, SEQ ID NO: 12, SEQ ID NO: 6, SEQ ID NO: 7, SEQ ID NO: 8, SEQ ID NO: 9, SEQ ID NO: 2, SEQ ID NO: 3, and SEQ ID NO: 4.
10. The method according to any one of items 1-9, wherein the arRNA is selected from the group consisting of: SEQ ID NO: 7, SEQ ID NO: 8, and SEQ ID NO: 9.
11. The method according to any one of items 1-10, wherein the construct is a linear nucleic acid, a viral vector, or a plasmid.
12. The method according to item 11, wherein the virus is an adeno-associated virus (AAV) or a lentivirus.
13. The method according to item 12, wherein the AAV vector is introduced into the cell by infection after being packaged into an AAV2, AAV5 or AAV8 capsid.
14. The method according to item 13, wherein the AAV vector is introduced into the cell by infection after being packaged into an AAV8 capsid.
15. The method according to any one of claims 1-14, wherein the cell is an optic nerve cell or auditory nerve cell of a mammalian.
16. An arRNA or its coding sequence for targeted editing of the target RNA containing a G to A mutation in a USH2A gene transcript, the arRNA comprises or consists of a sequence selected from the group consisting of: SEQ ID NO : 23, SEQ ID NO: 25, SEQ ID NO: 26, SEQ ID NO: 27, SEQ ID NO: 28, SEQ ID NO: 29, SEQ ID NO: 30, SEQ ID NO: 17, SEQ ID NO: 18 , SEQ ID NO: 19, SEQ ID NO: 11, SEQ ID NO: 12, SEQ ID NO: 6, SEQ ID NO: 7, SEQ ID NO: 8, SEQ ID NO: 9, SEQ ID NO: 2, SEQ ID NO: 3, and SEQ ID NO: 4.
17. A construct or delivery vector comprising the arRNA according to item 16.
18. The construct or delivery vector according to item 17, which is a plasmid, virus, liposome, or lipid nanoparticle.
19. The construct or delivery vector according to item 18, which is an adeno-associated virus (AAV) or a lentivirus.
20. The construct or delivery vector according to item 19, wherein the virus is AAV2, AAV5, or AAV8.
   In some embodiments, the application provides a composition, formulation, kit or biological product comprising the above arRNA, or comprising the above construct or delivery vector.
21. A cell obtained by the editing method according to any one of items 1-15.
22. A method of treating Usher type II syndrome in an individual, comprising: correcting a G to A mutation associated with Usher type II syndrome in a cell of the individual by the method according to any one of items 1-15.
23. The method according to item 22, comprising: introducing the arRNA according to item 16 or the construct or delivery vector according to any one of items 17-20 into a subject.
24. The method according to item 23, wherein the arRNA according to item 16 or the construct or delivery vector according to any one of items 17-20 is introduced into the subretinal space of the subject.

The method for targeted editing of target RNA transcribed in a cell containing NM_206933.2(USH2A)_c.11864G>A (p.Trp3955Ter) mutation sites on the basis of LEAPER technology provided by the present application has obvious advantages in the treatment of Usher syndrome type II:
Firstly, the LEAPER technology only needs to introduce short RNA, which can be limited within 151, 131, 121, or 111 nucleotides, and this makes the requirement on length of the introduced fragment far smaller than the upper limit of the delivery length of adeno-associated virus. Therefore, this technique is very suitable for the application of adeno-associated virus vectors as compared to direct introduction of the USH2A gene. Since eyes are an immune-privileged area and have very poor ability to clear adeno-associated virus, this makes the adeno-associated virus stay in the eye for quite long time. In addition, the functional cells are all nerve cells, and these cells do not divide under normal circumstances, so that the adeno-associated virus is allowed to stay in these cells for a long time, i.e., it is neither cleared by immunity nor diluted due to cell division. More importantly, compared with the previous common methods, the technical solution of the present application neither needs to truncate Usherin artificially nor to skip the mutated exons, so that a full-length normal Usherin can be obtained. Therefore, in theory, patients can get normal Usherin that is no different from ordinary people after being treated with this technology. Since USHER syndrome type II is a recessive genetic disease, this shows that normal functions can be obtained as long as some normal proteins can function. In this present application it is proved by experimental data that, the technical solution of the present application can correct USHER syndrome type II related gene transcripts (for example, the human USH2A gene transcript containing the NM_206933.2(USH2A)_c.11864 G>A (p.Trp3955Ter) mutation site), thereby achieving the purpose of the treatment of USHER syndrome type II.

### BRIEF DESCRIPTION OF THE DRAWINGS

Fig. 1 shows a schematic diagram of the progression of night blindness in Usher syndrome type II patients¹³.
Fig. 2 shows the distribution of pathogenic gene mutations in 138 Usher patients⁷.
Fig. 3 shows the analysis chart of USH2A gene mutation type⁷.
Fig. 4 shows a schematic diagram of the design of the reporting system reflecting the editing efficiency of the USH2A gene mutation site.
Fig. 5 shows a schematic diagram of the evaluation indexes of the editing efficiency of the USH2A gene mutation site.
Fig. 6 shows the test results of the editing efficiency of arRNAs with different length.
Fig. 7 shows the intensity of FITC channel for the false positive cells in the reporting system.
Fig. 8 shows the association between arRNA length and editing efficiency in LEAPER technology¹⁰ .
Fig. 9 shows the test results of the editing efficiency of arRNA truncated from 5' and 3' to the middle.
Fig. 10 shows the test results of the editing efficiency after fixing the length of the 3' end of arRNA to 25nt and truncating gradually from 5'.
Fig. 11 shows the effect of the position of targeting base on editing efficiency.

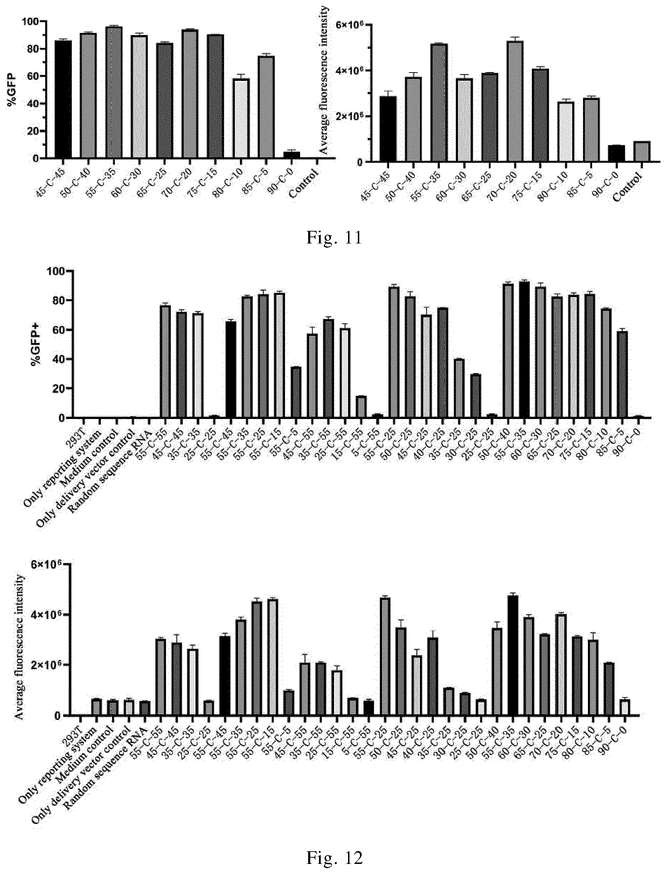

Fig. 12 shows a comparison of the results of repeated tests of arRNA editing efficiency of the same batch. In the figure, 293T represents the 293T that is not infected with the reporting system; the "only reporting system" is the 293T infected with the reporting system; Medium control (Opti-DMEM) is the blank control of the medium without RNAi MAX in the transfection; the "only delivery vector control" is the control only comprising RNAi MAX, and corresponding to the control (NC) wells in the previous test; the "random sequence RNA" is a 91 nt random RNA sequence that does not match with human genome 
(uaauccugaauaucgcgcaauuccccagcagagaacaucgcggugugaacgucccuuuauaccgggcagguauagcuga
aaucagcguggc (SEQ ID NO: 35)).
Fig. 13 shows a comparison of the results of repeated tests of editing efficiency obtained by next-generation sequencing of the same batch arRNA.
Fig. 14 shows the in vivo editing efficiency of arRNA delivered by AAV2 and AAV5 by intravitreal injection.
Fig. 15 shows the in vivo editing efficiency of arRNA delivered by AAV5 and AAV8 over time by subretinal injection.

### DETAILED DESCRIPTION OF THE INVENTION

This application aims at the mutation NM_206933.2(USH2A)_c.11864G>A (p.Trp3955Ter) with the highest proportion in the pathogenic gene USH2A of Usher syndrome type II, to create a new technical solution, i.e., under condition of without introducing oversized nucleic acid or exogenous protein molecule, precise editing of mutation site on the target RNA is performed by AAV, thereby restoring full functionality of Usherin protein, and continuously maintaining the function of Usherin protein for a long period of time.

In order to meet the above purpose, the present application provides: a method based on LEAPER technology for targeted editing of target RNA comprising NM_206933.2(USH2A)_c.11864G>A (p.Trp3955Ter) mutation site which is transcribed in a cell (hereinafter referred to as mutation editing method), an arRNA targeting the NM_206933.2(USH2A)_c.11864G>A (p.Trp3955Ter) mutation site, a construct comprising the arRNA, a cell prepared by the mutation editing method, a kit for editing a target RNA in a cell, a method for treating a disease caused by the NM_206933.2(USH2A)_c.11864G>A (p.Trp3955Ter) mutation in an individual (hereinafter referred to as a treatment method), and a formulation for use in the mutation editing method or the treatment method.

### Definition

RNA editing refers to a natural process that exists in eukaryotic cells. RNA editing is the editing of base A (adenine) into base I (hypoxanthine) that occurs at the RNA level after DNA transcription and before protein translation. Hypoxanthine (I) is recognized as G during translation, and the editing of A into I in RNA diversifies the transcriptome. The total amount of RNA is increased several-fold by site-specific and precise modification of the RNA molecule. This editing is catalyzed by ADAR (adenosine deaminase acting on RNA) protease, and is called site-directed RNA editing. This editing can occur in a coding region comprising intron and exon sequences, as well as in a non-coding region, and the editing of a coding region can redefine the coding sequence of a protein.

As used herein, "LEAPER technology", i.e., a technology for editing RNA by recruiting endogenous ADARs with engineered RNA, refers to RNA editing technology as reported in WO2020074001A1. The engineered RNA, i.e., adenosine deaminase recruiting RNA (arRNA), as used herein, refers to an RNA capable of deaminating a target adenosine in RNA by recruiting ADAR or certain complexes comprising an ADAR domain. arRNA is a kind of guide RNA. In this application, "guide RNA" refers to the modification of a target base by complementary hybridization with a target RNA and recruiting an enzyme that can modify the target base. The guide RNA includes arRNAs as well as gRNAs for other editing systems such as CRISPR.

As used herein, the term "adenosine deaminase (ADAR)" refers to a class of adenosine deaminase enzymes widely expressed in various tissues of eukaryotes (including mammals such as human), capable of catalyzing conversion of adenosine A into inosine I in RNA molecules. In the process of eukaryotic protein synthesis, I is usually translated as G.

As used herein, "complementarity" of nucleic acids refers to the ability of one nucleic acid chain to form hydrogen bonds with another nucleic acid chain through traditional Watson-Crick base pairing. Percent complementarity represents the percentage of residues in one nucleic acid molecule that can form hydrogen bonds (i.e., Watson-Crick base pairing) with another nucleic acid molecule (e.g., about 5, 6, 7, 8, 9, 10 out of 10 are respectively expressed as about 50%, 60%, 70%, 80%, 90% and 100% complementary). "Perfectly complementary" means that all contiguous residues of a nucleic acid sequence form hydrogen bonds with the same number of contiguous residues in a second nucleic acid sequence. As used herein, "substantially complementary" means the degree of complementarity of any of at least about 70%, 75%, 80% 85%, 90%, 95%, 97%, 98%, 99% or 100% over a region of about 40, 50, 60, 70, 80, 100, 150, 200, 250 or more nucleotides; or refers to two nucleic acids that hybridize under stringent conditions. For a single base or a single nucleotide, according to the Watson-Crick base pairing principle, when A is paired with T or U, C with G or I, it is called complementary or matched, and vice versa; and other base pairings are both called non-complementary or mismatched.

"Hybridization" refers to a reaction in which one or more polynucleotides react to form a complex stabilized by hydrogen bonding between the bases of nucleotide residues. Said hydrogen bonding can occur by Watson Crick base pairing, Hoogstein binding, or in any other sequence-specific manner. A sequence capable of hybridizing to a given sequence is referred to as the "complementary sequence" of the given sequence.

As used herein, the term "introduction" refers to the introduction of biomacromolecules such as nucleic acids and proteins into the cell membrane from outside the cell membrane by some means. The "introduction" includes spot transfection, lipofection, lipid-nanoparticle delivery, and the like.

As used herein, the term "electrotransfection" refers to the electroporation transfection technique, which temporarily forms small pores or openings in the cell membrane by applying an electric field to cells for a few microseconds to several milliseconds to deliver macromolecules such as DNA to a cell and eventually into the nucleus of the cell.

As used herein, the term "lipofection (Lipo)" refers to a transfection technique using liposomes as delivery vehicles in vivo and in vitro. Liposomes include neutral liposomes and cationic liposomes, wherein neutral liposomes use lipid membranes to encapsulate macromolecules, such as nucleic acids, thereby delivering the macromolecules into cell membranes by means of lipid membranes; cationic liposomes are positively charged, and the macromolecules transferred by them are not pre-embedded in them, but automatically bind to the positively charged liposomes due to the negative charge of the macromolecules themselves to form a macromolecule-cationic liposome complex, which adsorbs to the negatively charged cell membrane surface and is delivered into the cell via endocytosis.

As used herein, the term "lipid-nanoparticle (LNP) delivery" refers to transmembrane delivery of macromolecules, such as nucleic acid and protein, into cells via lipid nanoparticles. Among them, lipid nanoparticles refer to the pellets that are synthesized by mixing of two phases, which include ethanol phase containing ionic lipids, auxiliary phospholipids, cholesterol and PEG lipid, and acidic water phase containing large molecules such as nucleic acids and proteins. For example, LNP with RNA packed into it can enter the cytoplasm through endocytosis.

In this article, NM_206933.2(USH2A)_c.11864G>A (p.Trp3955Ter) mutation refers to a mutation corresponding to a change from G to A at position 11864 of NM_206933.2 (USH2A) gene transcript in human USH2A gene, the mutation leads to the transformation of the encoding sequence of tryptophan (Trp) at position 3955 of the peptide translated from the transcript to a termination codon, so that the amino acids of the final translation lack of all the amino acids after position 3955, thereby losing the protein activity of Usherin. Patients with this mutation will have the degenerative death of cone cells and rod cells, which eventually leads to blindness. The technical solutions of this application can restore the activity of Usherin protein by reverse this mutation at the transcription level. In some embodiments of this application, NM_206933.2 (USH2A)_c.11864G>A (p.Trp3955Ter) mutation site is also called USH2A pathogenic site.

As used herein, the term "target RNA" refers to the RNA of interest to be edited, which is transcribed from the USH2A gene and comprises a mutation from G to A. The target RNA can be a mature mRNA or mRNA precursor. In this application, mRNA precursor is more preferably. In some embodiments, the "target RNA" is an RNA containing NM_206933.2 (USH2A)_c.11864G>A (p.Trp3955Ter) mutation site. The A base formed by the transcription of the G to A mutation site in the "target RNA" is called "target adenosine" or "target A". The base corresponding to "target A" in arRNA is called "targeting base".

As used herein, the term "AAV" refers to "adeno-associated virus", or "adeno-associated virus vector". In this article, unless otherwise specified, "AAV", "adeno-associated virus" and "adeno-associated virus vector" can be used interchangeably. AAV belongs to microvirus family, which is a single-stranded linear DNA virus without envelope. Exogenous gene can be transferred into animal tissue and cells by using adeno-associated virus. The transferred exogenous gene can exist stably outside of the genes of the host genome and be expressed. There are many (12 kinds of) adeno-associated virus serotype, including for example: AAV1, AAV2, AAV5, AAV8, and AAV9 etc. As used herein, "patients" or "subjects" can be used interchangeably. In this article, unless otherwise specified, "patients" or "subjects" herein refer to human patients with NM_206933.2(USH2A)_c.11864G>A (p.Trp3955Ter) mutation in genome.

As used herein, the term "delivery" refers to the introduction of biomacromolecules such as nucleic acids and proteins into cell membrane from outside the cell membrane by some means. Said "delivery" refers to a delivery method such as electrotransfection, lipofection, lipid-nanoparticle delivery, virus delivery, exosome delivery, or the like. In this article, a combination of a biomacromolecule and a substance used for packaging the biomacromolecule or a substance connected to the biomacromolecule to promote the biomacromolecule to pass through the cell membrane and enter into a cell is called "delivery vector". For example, a liposomes, LNP, exosome, virus particle and the like in which a nucleic acid molecule is packaged by it.

As used herein, the term "construct" refers to a nucleic acid vector containing a specific nucleic acid sequence, which can be a linear nucleic acid molecule, plasmid or virus vector etc. The nucleic acid molecule may be a single-stranded or double-stranded molecule. The specific nucleic acid sequence can be a DNA sequence or a RNA sequence. In some embodiments, the nucleic acid sequence directly exerts its function without transcription, translation or expression. In some embodiments, the nucleic acid sequence is a DNA sequence, and exerts its function in form of RNA molecule after forming RNA through transcription. In some embodiments, the nucleic acid sequence is RNA, and exerts its function in form of polypeptide or protein after translation. In some embodiments, the nucleic acid sequence is DNA, and it exerts its function in form of protein after forming protein through transcription and translation steps. The construct can enter a target cell by packaging as a virus, lipid nanoparticle or exosome etc., or enter a target cell through electroporation, micro-injection, chemical transformation and other methods.

The term "modification" used herein refers to changing the composition or structure of a nucleic acid or protein through chemical or biological methods, such as genetic engineering methods, so as to change one or more features or functions of the nucleic acid or protein. In some embodiments of this application, the modification of arRNA makes it more stable after being introduced into a cell. In this article, the length of targeted base to 3 'end refers to the number of all bases from the nearest base at the 3' of the targeted base to the last base of the 3' end; the length of targeted base to 5 'end refers to the number of all bases from the nearest base at the 5' of the targeted base to the last base of the 5' end.

Unless otherwise defined in this article, all the technical and scientific terms used herein have the same meaning as that understood by the ordinary technical personnel in the field of the invention.

### Method for Editing Mutation

The present application provides a method for targeted editing of target RNA in a cell based on LEAPER technology, wherein the target RNA is an RNA containing G to A mutation in a USH2A gene transcript, and the method comprises:
introducing a construct comprising an adenosine deaminase recruiting RNA (arRNA) for editing the target RNA or a construct encoding the arRNA into the cell, wherein the arRNA comprises a complementary RNA sequence that hybridizes to the target RNA, and wherein the arRNA is capable of recruiting adenosine deaminase acting on RNA (ADAR), thereby the target adenosine in the target RNA is deaminated.

In some embodiments, the target RNA is mRNA or mRNA precursor (pre-mRNA), preferably, the target RNA is pre-mRNA.

In some embodiments, wherein the length of the arRNA is > 51 nt, preferably > 61 nt, more preferably > 65 nt. In some embodiments, the length of the arRNA is about 151-61 nt, 131-66 nt, 121-66 nt, 111-66 nt, 91-66 nt, or 81-66 nt. In some embodiments, the length of the arRNA is any natural number selected from 66nt to 131nt, such as 66nt, 67nt, 68nt, 69nt, 70nt, 71nt, 72nt, 73nt, 74nt, 75nt, 76nt, 78nt, 79nt, 80nt, 83nt, 85nt, 88nt, 91nt, 96nt, 98nt, 100nt, 105nt, 108nt, 100nt, 105nt, 110nt, 115nt, 120nt, 125nt, 130nt, etc. Under the condition that the transfection efficiency can be kept the same, preferably the length of the arRNA is about 71nt, for example, any natural number of nt from 66nt-76nt.

In some embodiments, the length from the targeting base to the 3' end in the arRNA is ≥5nt, such as ≥7nt, ≥10nt, ≥15nt, such as any natural number of nt selected from: 16-55nt, 20-50nt, 25-45nt, 25-35nt, or 25-30nt. In some embodiments, the length from the targeting base to the 5' end in the arRNA is ≥25nt, such as ≥30, ≥35, ≥45, such as any natural number of nt selected from: 46-90nt, 50-85nt, 55-80nt, 60-75nt, or 65-70nt.

In some embodiments, when the arRNA is complementarily hybridized to the target RNA, the preference order of targeting base of the arRNA corresponding to the target base on the target RNA from high to low is C, A, U, or G. That is, under normal circumstances, for the arRNA whose length and sequence other than the targeting base are the same, and the targeting base is respectively C, A, U, or G, the editing efficiency decreases sequentially. Therefore, most preferably, the targeting base of the arRNA is C, followed by A, then U, and then G.

In some embodiments, the target RNA is an RNA comprising a transcribed NM_206933.2(USH2A)_c.11864 G>A (p.Trp3955Ter) mutation site. In some embodiments, the arRNA is selected from the group consisting of: SEQ ID NO: 23, SEQ ID NO: 25, SEQ ID NO: 26, SEQ ID NO: 27, SEQ ID NO: 28, SEQ ID NO: 29, SEQ ID NO: 30, SEQ ID NO: 17, SEQ ID NO: 18, SEQ ID NO: 19, SEQ ID NO: 11, SEQ ID NO: 12, SEQ ID NO: 6, SEQ ID NO: 7, SEQ ID NO: 8, SEQ ID NO: 9, SEQ ID NO: 2, SEQ ID NO: 3, and SEQ ID NO: 4. In some embodiments, the arRNA is preferably selected from the group consisting of: SEQ ID NO: 7, SEQ ID NO: 8, and SEQ ID NO: 9; more preferably SEQ ID NO: 8, or SEQ ID NO: 9.

In some embodiments, the construct is selected from the group consisting of: a linear nucleic acid, a viral vector, and a plasmid. The linear nucleic acid includes: single-stranded nucleic acid, such as single-stranded RNA, or single-stranded DNA; and double-stranded nucleic acid, such as double-stranded DNA, or double-stranded RNA. In some embodiments, the linear nucleic acid can be chemically modified, such as 2'-O-Me modification and/or phosphorothioate bond modification. In some particular embodiments, the linear nucleic acid is chemically synthesized in vitro. In some particular embodiments, the linear nucleic acid is obtained by isolation and extraction after being synthesized by cells or organisms. In some embodiments, the construct is an adeno-associated virus (AAV) vector, or a lentiviral vector. In some embodiments, after being packaged into an AAV2, AAV5 or AAV8 capsid, the AAV vector is introduced into the cell by infection. In some embodiments, after being packaged into an AAV8 capsid, the AAV vector is introduced into the cell by infection. In some embodiments, the arRNA coding sequence is inserted into the cell genome by the construct for long-term expression. In some embodiments, the construct causes the arRNA coding sequence to be present and expressed outside the genome of the cell as episomal nucleic acid.

In some embodiments, the cells are eukaryotic cells. In some embodiments, the cells are mammalian cells. In some embodiments, the cells are nerve cells. In some embodiments, the nerve cells are sensory nerve cells. In some embodiments, the sensory nerve cells are selected from the group consisting of: optic nerve cells and auditory nerve cells. In some embodiments, the optic nerve cells are cone cells and/or rod cells.

In some embodiments, the introduction is electrotransfection, lipofection, lipid-nanoparticle delivery, or viral infection. In the embodiments of the present application, viral infection is preferred. Infectious viral particles, such as AAV or lentiviral particles, are formed, for example, by encapsulating an arRNA-encoding viral construct into a viral capsid, and the arRNA-encoding sequence is introduced into a cell by infection of the cell by the viral particle.

### Functional arRNA

The present application also provides an arRNA (hereinafter referred to as functional arRNA), the arRNA can be used for the aforementioned method for targeted editing of the target RNA in a cell based on the LEAPER technology. In some embodiments, the method is a method based on the LEAPER technology for targeted editing of the target RNA transcribed in a cell comprising the NM_206933.2(USH2A)_c.11864G>A (p.Trp3955Ter) mutation site. The arRNA comprises a complementary RNA sequence that can hybridize to the target RNA, and wherein the arRNA is capable of recruiting adenosine deaminase acting on RNA (ADAR), thereby the target adenosine in the target RNA is deaminated. In some embodiments, the target RNA targeted by the arRNA is selected from mRNA or Pre-mRNA, preferably Pre-mRNA.

In some embodiments, the length of the arRNA is >51nt, preferably >61nt, more preferably >65nt. In some embodiments, the length of the arRNA is about 151-61 nt, 131-66 nt, 121-66 nt, 111-66 nt, 91-66 nt, or 81-66 nt. In some embodiments, the length of the arRNA is any natural number selected from 66nt to 131nt, such as 66nt, 67nt, 68nt, 69nt, 70nt, 71nt, 72nt, 73nt, 74nt, 75nt, 76nt, 78nt, 79nt, 80nt, 83nt, 85nt, 88nt, 91nt, 96nt, 98nt, 100nt, 105nt, 108nt, 100nt, 105nt, 110nt, 115nt, 120nt, 125nt, 130nt, etc. Under the condition that the transfection efficiency can be kept the same, preferably, the length of the arRNA is about 71nt, for example: any natural number of nt selected from 66nt-76nt.

In some embodiments, the length from the targeting base to the 3' end in the arRNA is >5nt, such as >7nt, >10nt, >15nt, such as any natural number of nt selected from 16-55nt, 20-50nt, 25-45nt, 25-35nt, or 25-30nt. In some embodiments, the length from the targeting base to the 5' end in the arRNA is ≥25nt, such as, ≥30, ≥35, ≥45, e.g., any natural number of nt selected from 46-90nt, 50-85nt, 55-80nt, 60-75nt, or 65nt -70nt.

In some embodiments, when the arRNA is complementarily hybridized to the target RNA, the preference order of the targeting base of the arRNA corresponding to the target base on the target RNA is C, A, U, or G. That is, under normal circumstances, for the arRNA whose length and sequence other than the targeting base are the same, and the targeting base is respectively C, A, U, or G, the editing efficiency decreases sequentially. Therefore, most preferably, the targeting base of the arRNA is C, followed by A, then U, and then G.

In some embodiments, the arRNA is selected from the group consisting of: SEQ ID NO: 23, SEQ ID NO: 25, SEQ ID NO: 26, SEQ ID NO: 27, SEQ ID NO: 28, SEQ ID NO: 29, SEQ ID NO: 30, SEQ ID NO: 17, SEQ ID NO: 18, SEQ ID NO: 19, SEQ ID NO: 11, SEQ ID NO: 12, SEQ ID NO: 6, SEQ ID NO: 7, SEQ ID NO: 8, SEQ ID NO: 9, SEQ ID NO: 2, SEQ ID NO: 3, and SEQ ID NO: 4. Preferably, in some embodiments, the arRNA is selected from the group consisting of: SEQ ID NO: 7, SEQ ID NO: 8, and SEQ ID NO: 9, more preferably SEQ ID NO: 8 or SEQ ID NO: 9. In some embodiments, the editing efficiency of the targeting base which is introduced into the arRNA to paire with the target A in the target sequence is C, A, U, G in descending order,i.e., for the arRNA whose length and sequence other than the targeting base are the same, and the targeting base is respectively C, A, U, G, the editing efficiency decreases sequentially. Therefore, most preferably, the targeting base of the arRNA is C, followed by A, then U, and then G.

### Functional Construct

This application also provides a construct encoding arRNA (hereinafter referred to as a functional construct), wherein the functional construct can be used for the aforementioned method for targeted editing of target RNA in a cell based on the LEAPER technology. In some embodiments, the method is a method based on the LEAPER technology for targeted editing of target RNAin a cell which comprises a transcribed NM_206933.2(USH2A)_c.11864G>A (p.Trp3955Ter) mutation site; wherein the arRNA contains the aforementioned functional arRNA.

In some embodiments, the construct is selected from the group consisting of: linear nucleic acid, virus vector, or plasmid. The linear nucleic acid includes single-stranded nucleic acid, such as single-stranded RNA, or single-stranded DNA; and double-stranded nucleic acid, such as double-stranded DNA, or double-stranded RNA. In some embodiments, the linear nucleic acid can be chemically modified, such as 2'-O-ME modification and/or phosphorothioate bond modification. In some particular embodiments, the linear nucleic acid is chemically synthesized in vitro. In some particular embodiments, the linear nucleic acid is obtained by isolation and extraction after being synthesized by cells or organisms. In some embodiments, the construct is an adeno-associated virus (AAV) vector, or a lentiviral vector. In some embodiments, the AAV vector is packaged into an AAV2, AAV5 or AAV8 capsid to form a virus particle that can infect a cell, thereby the AAV vector is introduced into the cell through the infection of the cell by the virus particle. In some embodiments, preferably the construct is packaged into AAV8. In some embodiments, the arRNA coding sequence is inserted into the cell genome by the construct for long-term expression. In some embodiments, the construct causes the arRNA coding sequence to be present and expressed outside the genome of the cell as episomal nucleic acid.

This application provides a construct containing the aforementioned functional arRNA, wherein the construct is selected from the group consisting of: linear nucleic acid, plasmid, and virus vector. This application also provides a delivery vector comprising the construct, and the delivery vector is selected from the group consisting of: a virus particle, liposome, lipid nanoparticle and exosome.

### Cell

This application also provides a cell, which can be edited and prepared by the aforementioned method for targeted editing of target RNA in a cell based on the LEAPER technology. In some embodiments, the cell is a cell from a patient with NM_206933.2(USH2A)_c.11864G>A (p.Trp3955Ter) mutation site, the target base A in the target RNA transcribed from the mutation site can be deaminated to form base I by the aforementioned mutation editing method, the "I" will be recognized as G in the subsequent translation process to restore the complete function of Usherin protein. In some embodiments, the patient's cells are stem cells or induced multi-potential stem cells, the stem cells or cells differentiated from the stem cells by induction can be transplanted to a specific location in a patient. In some embodiments, the stem cells can be transplanted back to a specific location in the patient's eyes to exert function after inducing and being differentiated into a healthy cone and/or visual rod cells in vitro.

### Kit

This application also provides a kit for editing a target RNA in a cell, which contains the aforementioned functional arRNA, or the aforementioned functional construct for encoding arRNA. In some embodiments, the kit comprises nucleic acid, plasmid, genome, and cells, etc. that can be used for editing NM_206933.2(USH2A)_c.11864G>A (p.Trp3955Ter) mutation. In some embodiments, the kit also further comprises a solvent that is suitable for dissolving the functional arRNA or the functional construct. In some embodiments, the kit also further comprises instructions to inform a user the various ingredients and the contents thereof which are comprised in the kit, and/or the method for using the kit.

### Treatment Method

This application also provides a method for treating a disease in an individual caused by NM_206933.2(USH2A)_c.11864G>A (p.Trp3955Ter) mutation (hereinafter referred to as treatment method), which comprises: correcting a mutation in the target RNA in individual cells which is associated with the NM_206933.2(USH2A)_c.11864G>A (p.Trp3955Ter) mutation site by using the aforementioned method based on LEAPER technology for targeted editing of target RNA in a cell which comprises a transcribed NM_206933.2(USH2A)_c.11864G>A (p.Trp3955Ter) mutation site. In some embodiments, the disease includes USHER II syndrome.

In some embodiments, the treatment method comprises: injecting an arRNA for editing target RNA or a construct encoding the arRNA into the subretinal space or vitreum of the subject. In some preferable embodiments, the treatment method comprises: injecting an arRNA for editing target RNA or a construct encoding the arRNA into the subretinal space of the subject. In some embodiments, in the treatment only one injection is needed for the subject. In some embodiments, in the treatment injections at regular interval are needed for the subject. In some particular embodiments, in the treatment injections at an interval of two or more months are needed for the subject, for example, injections at an interval of one year, 10 years, 20 years, or 30 years et al. are needed for the subject.

### Formulation

This application also further provides a formulation, which comprises the aforementioned functional arRNA, or the aforementioned functional construct encoding the arRNA or a delivery vector thereof. In some embodiments, the formulation comprises the functional arRNA, or the functional construct or its delivery vector, and a transfection reagent. In some particular embodiments, the functional arRNA or functional construct is packaged in a lipidosome. In some particular embodiments, the functional arRNA or functional construct is prepared to form lipid nanoparticles. In some embodiments, the functional construct comprised in the formulation is a virus vector, such as AAV or lentiviral vector, the formulation comprises a living virus or virus lyophilized powder. The formulation thus further comprises a suitable stabilizer such as human albumin, gelatin, sucrose, etc.

The preferred implementations of the present invention are described above in detail, but the present invention is not limited to these. Within the scope of the technical conception of the present invention, a variety of simple modifications can be made based on the technical solutions of the present invention, including the combinations of each technical characteristic in any other appropriate way. These simple modifications and combinations should also be regarded as the content disclosed by the present invention, and all belong to the protection scope of the present invention. The technical solutions of the present invention will be described in further detail below with reference to particular Examples, but the present invention is not limited to the following Examples. Unless otherwise specified, the reagents mentioned below are all commercially available. For the sake of brevity, in some operations the operation parameters, steps and used instruments are not described in detail, and it should be understood that these are well known and reproducible by those skilled in the art.

### EXAMPLES

### Example 1: Construction of the Reporting System

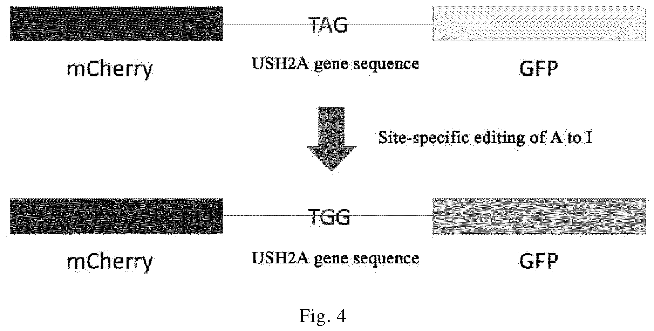

This application mainly studies the repair of NM_206933.2(USH2A)_c.11864G>A (p.Trp3955Ter) mutation site by LEAPER technology. Therefore, a simple and usable reporting system that can reflect the editing efficiency of this site is needed. Since the mutation from the original TGG codon to TAG codon caused by the pathogenic mutation site NM_206933.2(USH2A)_c.11864G>A (p.Trp3955Ter) in the USH2A gene is a nonsense mutation, the mutated mRNA will stop at the newly formed TAG stop codon during translation, and as a result the subsequent sequence cannot continue to be translated into proteins. Taking advantage of the above features, the present application designed a reporting system as shown in FIG. 4. The reporting system uses a lentiviral vector, and the mRNA shown in Fig. 4 is driven by a CMV promoter. This fragment of mRNA mainly comprises the following parts: 1) mCherry red fluorescent protein sequence, which will be stably expressed and will be translated normally whether it is edited or not, thus the red fluorescence intensity can be used as an internal reference; 2) USH2A gene mutation site and its adjacent 100 base pairs on each side, this region is a disease-related sequence; the sequence of the above part 2) is taken from positions 12203-12403 of the NM_206933.2 sequence, totally 201 base pairs, after a point mutation from G to A at the USH2A c. 11864 site, there is a TAG stop codon in the reading frame (in-frame) in this sequence, thus the translation will be stopped here during the translation process; 3) GFP (green fluorescent protein) sequence, the protein can emit green fluorescence, and the fluorescence intensity can be detected by the FITC channel in the flow cytometer (because the GFP sequence is in the same reading frame as the USH2A-related sequence and is located downstream thereof, if the TAG stop codon in the USH2A-related sequence is not edited by LEAPER technology, then the TAG stop codon will cause the subsequent GFP to fail to be translated normally, and no green fluorescence will show up; if the TAG stop codon in the USH2A-related sequence is successfully edited by the LEAPER technology to form a TIG codon, the stop codon will disappear, the subsequent GFP translation continues, and the green fluorescence will show up; 4) P2A and T2A sequences are inserted downstream of mCherry and upstream of GFP, during translation, one of the peptide bonds of these two sequences cannot be formed normally due to steric hindrance, thus the three portions of mCherry, the 201 base pairs of the middle sequence which is associated with USH2A, and GFP are separated during the translation process, so that the USH2A-related sequence inserted in the middle will not affect the functions of mCherry and GFP.

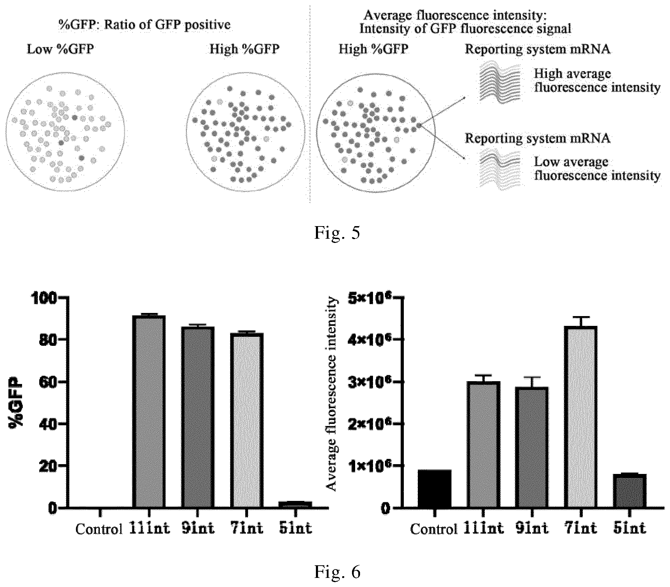

The entire sequence of the above sequence is synthesized in vitro through conventional technologies in the art. The specific sequence is shown in SEQ ID NO: 1, this sequence is cloned into the pCDH-CMV vector through the multiple cloning sites after the CMV promoter. The pCDH-CMV vector plasmid was a kind gift of Kazuhiro Oka (Addgene plasmid # 72265; http://n2t.net/addgene:72265; RRID: Addgene_72265). The constructed plasmid is packaged into lentivirus through the next-generation lentivirus packaging system (pCAG-VSVG was a kind gift of Arthur Nienhuis & Patrick Salmon, Addgene plasmid # 35616; http://n2t.net/addgene:35616; RRID:Addgene_35616; pCMVR8.74 was a kind gift of Didier Trono, Addgene plasmid # 22036; http://n2t.net/addgene:22036; RRID:Addgene_22036), and is used to infect 293T cells. 48h after the infection, the mCherry-positive cell population is sorted by a flow cytometry, and cells of this cell population are final cells of the reporting system, wherein the fluorescent threshold is adjusted until the cell number of the uninfected 293T cells or the cells of the untreated control reporting system whose fluorescence intensity is higher than the threshold is less than 1% (close to 1% as much as possible), at this time, the cells whose fluorescence intensity is higher than the fluorescent threshold are considered as positive cells. For the test of the reporting system, there are usually two evaluation indicators as shown in Fig. 5: 1. Ratio of GFP-positive cells (shortened as %GFP); 2. mean fluorescence intensity (MFI) of GFP+ cells. Among them, the proportion of GFP-positive cells (i.e., the intensity of GFP) higher than the proportion of the cells of the GFP positive threshold defined in this application. The level of %GFP represents the number of the edited cells. Among GFP-positive cells, due to the different fluorescence intensity of different cells, the test of the editing efficiency of the reporting system further includes MFI. Since cells in each well have similar red fluorescence intensity as an internal reference, it is considered that the higher the GFP% and MFI, the higher the degree of editing for the USH2A pathogenic mutation site of the cells in the well under the test conditions.

SEQ ID NO: 1: the sequence in lowercase letter indicates disease-related sequence, while the capital letter or lowercase letter of the same base does not represent different base type.

ATGGTGAGCAAGGGCGAGGAGGATAACATGGCCATCATCAAGGAGTTCATGCGCTTCAA
GGTGCACATGGAGGGCTCCGTGAACGGCCACGAGTTCGAGATCGAGGGCGAGGGCGAGGGCC
GCCCCTACGAGGGCACCCAGACCGCCAAGCTGAAGGTGACCAAGGGTGGCCCCCTGCCCTTC
GCCTGGGACATCCTGTCCCCTCAGTTCATGTACGGCTCCAAGGCCTACGTGAAGCACCCCGCCG
ACATCCCCGACTACTTGAAGCTGTCCTTCCCCGAGGGCTTCAAGTGGGAGCGCGTGATGAACT

TCGAGGACGGCGGCGTGGTGACCGTGACCCAGGACTCCTCCCTGCAGGACGGCGAGTTCATCT
ACAAGGTGAAGCTGCGCGGCACCAACTTCCCCTCCGACGGCCCCGTAATGCAGAAGAAGACC
ATGGGCTGGGAGGCCTCCTCCGAGCGGATGTACCCCGAGGACGGCGCCCTGAAGGGCGAGATC
AAGCAGAGGCTGAAGCTGAAGGACGGCGGCCACTACGACGCTGAGGTCAAGACCACCTACAA
GGCCAAGAAGCCCGTGCAGCTGCCCGGCGCCTACAACGTCAACATCAAGTTGGACATCACCTC
CCACAACGAGGACTACACCATCGTGGAACAGTACGAACGCGCCGAGGGCCGCCACTCCACCG
GCGGCATGGACGAGCTGTACAAGGCCACGAACTTCTCTCTGTTAAAGCAAGCAGGAGACGTGG
AAGAAAACCCCGGTCCTGCTAGCgcccttgaatttatggatgaaggagacaccctgaggcctttcacactctacgaatatcgggtcag
agcctgtaactccaagggttcagtggagagtctgtggtcattaacacaaactctggaagctccacctcaagattttccagctccttgggctcaagccacgagt
gctcattcagttctgttgaattggacaaagccaGCGGCCGCTGAGGGCAGAGGAAGTCTTCTAACATGCGGTGACG
TGGAGGAGAATCCCGGCCCTTCCGGAATGGAGAGCGACGAGAGCGGCCTGCCCGCCATGGAG
ATCGAGTGCCGCATCACCGGCACCCTGAACGGCGTGGAGTTCGAGCTGGTGGGCGGCGGAGA
GGGCACCCCCAAGCAGGGCCGCATGACCAACAAGATGAAGAGCACCAAAGGCGCCCTGACCT
TCAGCCCCTACCTGCTGAGCCACGTGATGGGCTACGGCTTCTACCACTTCGGCACCTACCCCAG
CGGCTACGAGAACCCCTTCCTGCACGCCATCAACAACGGCGGCTACACCAACACCCGCATCGA
GAAGTACGAGGACGGCGGCGTGCTGCACGTGAGCTTCAGCTACCGCTACGAGGCCGGCCGCG
TGATCGGCGACTTCAAGGTGGTGGGCACCGGCTTCCCCGAGGACAGCGTGATCTTCACCGACA
AGATCATCCGCAGCAACGCCACCGTGGAGCACCTGCACCCCATGGGCGATAACGTGCTGGTGG
GCAGCTTCGCCCGCACCTTCAGCCTGCGCGACGGCGGCTACTACAGCTTCGTGGTGGACAGCC
ACATGCACTTCAAGAGCGCCATCCACCCCAGCATCCTGCAGAACGGGGGCCCCATGTTCGCCTT
CCGCCGCGTGGAGGAGCTGCACAGCAACACCGAGCTGGGCATCGTGGAGTACCAGCACGCCT
TCAAGACCCCCATCGCCTTCGCC

### Example 2: arRNA Optimization Aiming at Pathogenic Mutation Site

Since LEAPER technology does not need to introduce any exogenous protein into a cell, only arRNA is needed to be introduced into the reporting system cell in this example when testing LEAPER's editing to the pathogenic site. In this example the arRNA is synthesized in vitro, and the sequences are shown in Table 1 below. These sequences are named in two ways, particularly the "name" is named according to the length of the sequences or the short cut length, and the "unified name" is named in X-C-Y format, wherein the "X" represents the length of the 5' end, the "Y" represents the length of the 3' end, and the "C" represents the targeting base C. For example, 111nt is named as 55-C-55, which means that the length of the 5' end and 3' end are both 55nt.

**Table 1: Summary of the arRNA sequences used in Example 2**

| Name | Unified name | RNA sequence |
|---|---|---|
| 111nt | 55-C-55 | SEQ ID NO: 2 : |
| | | agcccaaggagcuggaaaaucuugagguggagcuuccagaguuuguguuaaugaccacagacucuccacug aacccuuggaguuacaggcucugacccgauauucguagag |
| 91nt | 45-C-45 | SEQ ID NO: 3 : |
| | | gcuggaaaaucuugagguggagcuuccagaguuuguguuaaugaccacagacucuccacugaacccuugga guuacaggcucugacccgau |
| 71nt | 35-C-35 | SEQ ID NO: 4 : |
| | | cuugagguggagcuuccagaguuuguguuaaugaccacagacucuccacugaacccuuggaguuacaggcu |
| 51nt | 25-C-25 | SEQ ID NO: 5 : agcuuccagaguuuguguuaaugaccacagacucuccacugaacccuugga |
| 3-10 | 55-C-45 | SEQ ID NO: 6 : |
| | | agcccaaggagcuggaaaaucuugagguggagcuuccagaguuuguguuaaugaccacagacucuccacug aacccuuggaguuacaggcucugacccgau |
| 3-20 | 55-C-35 | SEQ ID NO: 7 : |
| | | agcccaaggagcuggaaaaucuugagguggagcuuccagaguuuguguuaaugaccacagacucuccacug aacccuuggaguuacaggcu |
| 3-30 | 55-C-25 | SEQ ID NO: 8 : |
| | | agcccaaggagcuggaaaaucuugagguggagcuuccagaguuuguguuaaugaccacagacucuccacug aacccuugga |
| 3-40 | 55-C-15 | SEQ ID NO: 9 : |
| | | agcccaaggagcuggaaaaucuugagguggagcuuccagaguuuguguuaaugaccacagacucuccacug |
| 3-50 | 55-C-5 | SEQ ID NO: 10 : |
| | | agcccaaggagcuggaaaaucuugagguggagcuuccagaguuuguguuaaugaccacaga |
| 5-10 | 45-C-55 | SEQ ID NO: 11 : |
| | | gcuggaaaaucuugagguggagcuuccagaguuuguguuaaugaccacagacucuccacugaacccuugga guuacaggcucugacccgauauucguagag |
| 5-20 | 35-C-55 | SEQ ID NO: 12 : |
| | | cuugagguggagcuuccagaguuuguguuaaugaccacagacucuccacugaacccuuggaguuacaggcu cugacccgauauucguagag |
| 5-30 | 25-C-55 | SEQ ID NO: 13 : |
| | | agcuuccagaguuuguguuaaugaccacagacucuccacugaacccuuggaguuacaggcucugacccgau auucguagag |
| 5-40 | 15-C-55 | SEQ ID NO: 14 : |
| | | guuuguguuaaugaccacagacucuccacugaacccuuggaguuacaggcucugacccgauauucguagag |
| 5-50 | 5-C-55 | SEQ ID NO: 15 : |
| | | augaccacagacucuccacugaacccuuggaguuacaggcucugacccgauauucguagag |
| 50-C-25 | 50-C-25 | SEQ ID NO: 17 : |
| | | aaggagcuggaaaaucuugagguggagcuuccagaguuuguguuaaugaccacagacucuccacugaaccc uugga |
| 45-C-25 | 45-C-25 | SEQ ID NO: 18 : |
| | | gcuggaaaaucuugagguggagcuuccagaguuuguguuaaugaccacagacucuccacugaacccuugga |
| 40-C-25 | 40-C-25 | SEQ ID NO: 19 : |
| | | aaaaucuugagguggagcuuccagaguuuguguuaaugaccacagacucuccacugaacccuugga |
| 35-C-25 | 35-C-25 | SEQ ID NO: 20 : |
| | | cuugagguggagcuuccagaguuuguguuaaugaccacagacucuccacugaacccuugga |
| 30-C-25 | 30-C-25 | SEQ ID NO: 21 : |
| 50-C-40 | 50-C-40 | gguggagcuuccagaguuuguguuaaugaccacagacucuccacugaacccuugga |
| | | SEQ ID NO: 23 : |
| | | aaggagcuggaaaaucuugagguggagcuuccagaguuuguguuaaugacCacagacucuccacugaacc cuuggaguuacaggcucugac |
| 60-C-30 | 60-C-30 | SEQ ID NO: 25 : |
| | | gcuugagcccaaggagcuggaaaaucuugagguggagcuuccagaguuuguguuaaugacCacagacucu ccacugaacccuuggaguuac |
| 65-C-25 | 65-C-25 | SEQ ID NO: 26 : |
| | | ucguggcuugagcccaaggagcuggaaaaucuugagguggagcuuccagaguuuguguuaaugacCacag acucuccacugaacccuugga |
| 70-C-20 | 70-C-20 | SEQ ID NO: 27 : |
| | | agcacucguggcuugagcccaaggagcuggaaaaucuugagguggagcuuccagaguuuguguuaaugac Cacagacucuccacugaaccc |
| 75-C-15 | 75-C-15 | SEQ ID NO: 28 : |
| | | gaaugagcacucguggcuugagcccaaggagcuggaaaaucuugagguggagcuuccagaguuuguguua augacCacagacucuccacug |
| 80-C-10 | 80-C-10 | SEQ ID NO: 29 : |
| | | gaacugaaugagcacucguggcuugagcccaaggagcuggaaaaucuugagguggagcuuccagaguuug uguuaaugacCacagacucuc |
| 85-C-5 | 85-C-5 | SEQ ID NO: 30 : |
| | | caacagaacugaaugagcacucguggcuugagcccaaggagcuggaaaaucuugagguggagcuuccagag uuuguguuaaugacCacaga |
| 90-C-0 | 90-C-0 | SEQ ID NO: 31 : |
| | | caauucaacagaacugaaugagcacucguggcuugagcccaaggagcuggaaaaucuugagguggagcuuc cagaguuuguguuaaugacC |

| | | |
|---|---|---|
| Note: The capital letters in the RNA sequence are only used to distinguish the targeting base, capital letter or lowercase letter of the same base does not represent different base type. | | |

As for all tests in this example, the RNAi MAX reagent (Invitrogen 13778150) is used to introduce arRNA into the cells. The specific steps are as follows:
1. DMEM (Hyclone SH30243.01) containing 10% FBS (Vistech SE100-011)s is used for cell culture. 12-well plate is used to cultivate cells, 15,000 cells/well for the cells in the reporting system. The moment of cell plating is recorded as 0 hour.
2. 24 hours after cell passaging, RNAi MAX reagent is used to transfer 12.5pmol of arRNA into each well. Please refer to the supplier's instructions for the transfection steps.
3. 72 hours after cell passaging, cells in the whole well are digested by using trypsin (Invitrogen 25300054), and the fluorescence intensity of the cells is analyzed in the FITC channel with a flow cytometer.

Firstly, arRNA is designed following the design principles of arRNA in references related to LEAPER (Leveraging Endogenous ADAR for Programmable Editing of RNA, Qu et al., 2019, as for the arRNA synthesized in vitro in this literature, only arRNA with the length of 111nt is used for testing), i.e., the counter position targeting base of base A (target A) at the pathogenic mutation site is identified as C, the sequence lengths at the 5' end and the 3' end of the targeted base are made the same. Based on the arRNA with the total length of 111nt (55-C-55) (consistent with the reference), the 5' end and 3' end are truncated concurrently to form the various testing arRNAs used in this application. Due to the limitation of in vitro synthetic RNA, it is hard to synthesize longer RNA in vitro at current stage. Therefore, only four arRNAs with the lengths of 111nt, 91nt, 71nt and 51nt are tested in this example, and the results of editing efficiency are shown in Fig. 6.

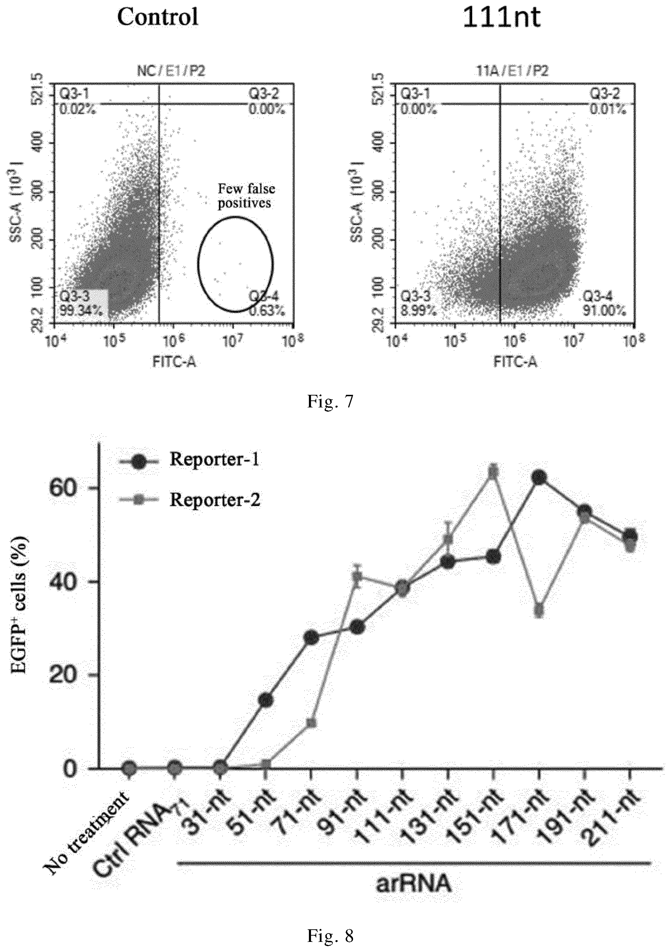

Particularly, "control" means the control well without any arRNA. It can be seen from Fig. 6 that, the GFP positive cells in the control well as background are very few (about 0.6%), in contrast, %GFP caused by the three arRNAs of 111nt, 91nt and 71nt all exceeds 90%, while there is only very low positive ratio for 51nt. It can be seen from the plot of average fluorescence intensity that, there are a small amount of fake-positive cells in the control well as background, and although these cells account for a very small proportion, their fluorescence intensity is high, as shown in Fig. 7.

Surprisingly, unlike those reported in above LEAPER references, this application finds that 51-111nt arRNA may achieve preferable editing result, however it is not that longer arRNAs are more efficient in editing. As shown in Fig. 8, according to those reported in the above LEAPER reference (the same as Fig. 2d of Qu et al., 2019), comparison between the 51-111nt arRNAs is conducted in its reporting system. Particularly, it can be seen that the proportion of GFP+ cells of 71nt is lower than that of 111nt, the relationship of length vs. editing efficiency lies in that the editing efficiency is basically increasing from the 51nt to 111nt. In the test of the reporting system in Example 1, the proportion of GFP positive cells caused by 71nt is not much different from that of 111nt, while the average fluorescence intensity of 71nt is higher than that of 111nt.

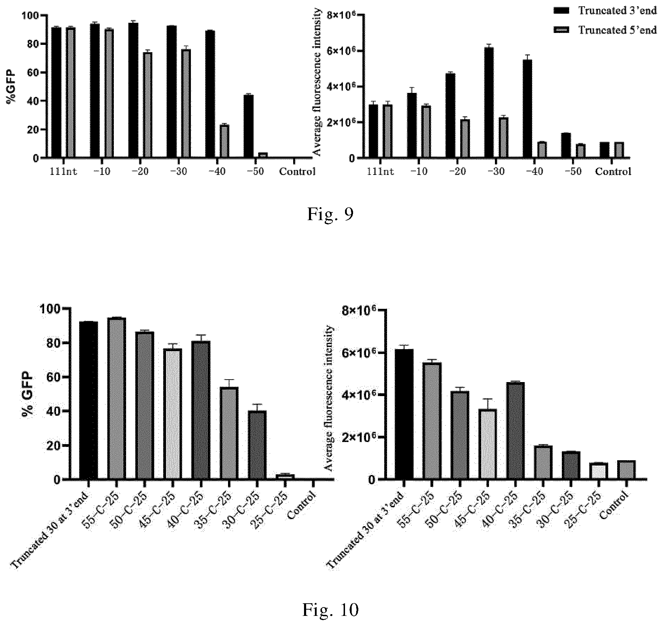

Secondly, since longer arRNAs are more expensive to synthesize, and longer arRNAs may lead to greater off-target potential, the editing efficiency of arRNAs truncated by various means is tested in this application. As shown in Fig. 9, the targeting base C is set as the midpoint, based on the 111nt arRNA sequence (SEQ ID NO: 2), it is gradually truncated to the middle from the upstream of the 5' end or the downstream of the 3' end in a step of 10 nt to obtain arRNA for testing.

It can be seen from Fig. 9 that, when truncated from the 5' end, the result is consistent with the report in the LEAPER reference, i.e., the editing efficiency of relatively longer RNAs is higher; however, what is different is that when truncated from the 3' end, the editing efficiency does not gradually decrease, on the contrary the highest editing efficiency appears when the 3' end is truncated by 30 nt. Since the highest editing efficiency appears when 30nt is truncated from the 3' end, and the length of the arRNA at this moment is: 55-C-25, therefore, in the following experiments, we set the length of the 3' end to 25nt, and continuously perform truncation from the 5' end to obtain the test arRNA, the results are shown in Fig. 10.

"3'-truncated 30" and "55-C-25" in Fig. 10, and "-30" of "truncated 3' end" in Fig. 9 are the same arRNA sequences (SEQ ID NO: 8), wherein "3'-truncated 30" in Fig. 10 and "-30" of "truncated 3' end" in Fig. 9 are synthesized in the same batch, and they are different from the synthetic batch of "55-C-25". It can be seen from Fig. 10 that, when the 3' end is fixed at 25nt and the 5' end is truncated, the editing efficiency basically shows a downward trend with the length decrease of the 5'. Although 40-C-25 does not conform to the above trend, neither its MFI nor the %GFP exceeds that of 55-C-25.

Finally, besides length, the position of the targeting base also has an impact on editing efficiency. Therefore, we then fix the length of the arRNA to 91nt, and the test arRNA is obtained by moving the position of the targeting base. The results are shown in Fig. 11.

It can be seen from Fig. 11 that, the highest editing efficiency does not appear when the targeting base is in the middle of the arRNA, and the arRNA has high editing efficiency before the length of the 3' end is reduced to 0 (as shown by 85-C-5 and its left column).

Finally, we synthesize all the above arRNAs in the same batch, and re-test them in order to eliminate the test errors caused by different batches. The results are shown in Fig. 12. It can be seen from Fig. 12 that, the three most efficient arRNAs are 55-C-35 (SEQ ID NO: 7), 55-C-25 (SEQ ID NO: 8), 55-C-15 (SEQ ID NO: 9). In the repeated tests, the overall editing efficiency is slightly decreased compared to the first test, possibly due to freezing and thawing of the arRNA used in the test. For bisymmetric arRNAs, editing efficiency of 71nt (35-C-35) is comparable to that of 111nt (55-C-55). In addition, the editing efficiency of the longest 111nt arRNA in this batch is not the highest. In contrast, the three arRNAs of 55-C-15, 55-C-25, and 55-C-35 with respective length of 71nt, 81nt, and 91nt have relatively higher editing efficiency.

Moreover, compared with the preferred 111nt arRNA in the prior art, the arRNA with a length of only 71nt such as 55-C-15 in this example not only has higher editing efficiency, but also has a lower synthesis cost (see Table 2 for the reference prices). Furthermore, it can be expected that shorter arRNAs may have a lower risk of off-target.

**Table 2: Synthesis length and price of arRNA**

| arRNA length | Name of arRNA | synthesis amount | reference price of synthesis |
|---|---|---|---|
| 111nt | 55-C-55 | 3nmol | $750 |
| 71nt | 55-C-15 | 5nmol | $314 |

By reading the GFP signal, we can quickly and roughly judge the editing efficiency of different arRNAs, but if the editing efficiency is to be confirmed more accurately, Next Generation Sequencing (NGS) is needed to finally confirm the ratio of the A in the mRNA that is edited into I (G).

Cell plating and transfection are performed according to the same steps 1 and 2 above in this example, 800 µL of TRIzol (Invitrogen, 15596018) is used to collect samples 72h after cell passaging, and the Direct-zol RNA Miniprep Kit (Zymo Resaerch, R2052) is used for RNA extraction. 1000ng of extracted RNA is taken from each sample, and reverse transcription is performed by TransScript^{®} One-Step gDNA Removal and cDNA Synthesis SuperMix Kit (TransGen Biotech, AT311) to synthesize cDNA. Afterwards, 1 µL of the reverse transcription product is taken to perform PCR with two primers with the sequences of ggagtgagtacggtgtgcGGAAGAAAACCCCGGTCCTGCTA (SEQ ID NO: 33) and gagttggatgctggatggAACAGAACTGAATGAGCACTCGTGG (SEQ ID NO: 34), and Q5 hot-start enzyme (NEB, M0494L). The PCR products are used to build library by using Hi-TOM kit (Novogene, REF PT045), and the next-generation sequencing and data analysis are completed according to the following steps.

### i. Illumina Sequencing

High-throughput sequencing is performed for the constructed sequencing library in PE150 mode through NovaSeq6000 platform.

### ii. Sequencing Data Processing

The quality-control of the raw data obtained by high-throughput sequencing by fastp (v0.19.6), and low-quality sequences, sequences with adapters, and sequences comprising polyG are filtered out. The obtained high-quality sequencing data is divided into each sample according to the corresponding barcode sequence, and alignment is performed with the sequence of the amplified target region by using BWA (v0.7.17-r1188) software, and format conversion is conduct to generate BAM files through SAMtools (v1.9). The alignment information is counted, and the order is rearranged to build indexes.

### iii. Editing Efficiency Analysis

All potential RNA mutation sites are detected by using JACUSA (v1.3.0) software with the parameters: call-1 -a B,R,D,I,Y,M:4 -C ACGT -c 2 -p 1 -P UNSTRANDED -R -u DirMult-CE. After filtering out high-frequency point mutations appeared both in the control and treated samples, three times of the average mutation frequency outside the A->G mutation site is used as the threshold, the part of the target base A->G mutation frequency above the threshold is taken as the real frequency of target A to G mutation.

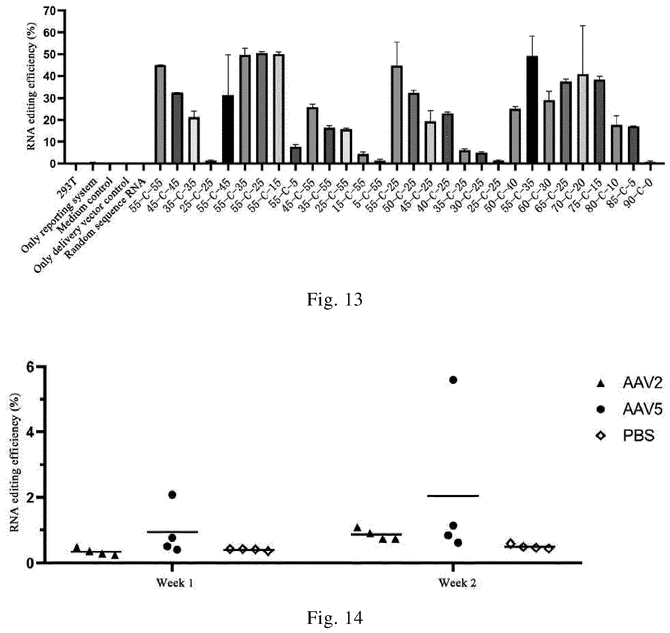

Using the above steps, we finally perform next-generation sequencing on all the samples in Fig. 12, the editing efficiency is taken as the percentage of the number of the Reads whose target base is G to the total number of the Reads whose target base is ATCG, plotting on the basis of the editing efficiency. The result is shown in the Fig. 13.

It can be seen from the comparison of Fig. 12 and Fig. 13 that, the editing efficiency obtained by different methods shows basically the same trend. That is, the 111nt arRNA designed according to the LEAPER literature (Qu et al., 2019) is not the arRNA with the highest editing efficiency, however, after the truncation of the 3' end and the 5' end of the targeting base and the adjustment of the position of the targeting base in the sequence, it is finally found that the sequence of 55-C-15 (SEQ ID NO: 9) not only shows a high editing efficiency on the read value of GFP, but can also it can be further confirmed by next-generation sequencing, the editing efficiency of A to G can reach about 50%. This means that the editing of A to G occurs at 50% of the target base sites on the mRNA after editing with 55-C-15 arRNA.

Through the above experiments, we confirm that the arRNA designed according to the technical solution described in this application may achieve higher editing efficiency and lower cost than the prior art. At the same time, it can be predicted that since the required length of the arRNA of the present application is shorter, under the condition of the same number of transfection copies, the mass of the arRNA transfected into cells in this application is lower, this will be beneficial to reduce the toxicity to cells caused by the transfer of excess RNA, and also reduce the binding of arRNA to non-target RNA sequences, thereby improving the safety of in vivo editing.

### Example 3: In Vivo Editing of the Ush2A Gene Site at Mouse Eye

In Example 2, the USH2A pathogenic site is edited by in vitro experiments. Although good editing efficiency can be achieves in vitro experiments, the environment of in vitro cell culture cannot simulate many factors such as human circulation, immune system, and intraocular injection, and these still need to be verified by more in vivo experiments. Considering the follow-up connection with actual therapy, currently adeno-associated virus (AAV) is usually used for delivery of small RNAs to eyes, we finally chose to use AAV vectors to deliver arRNA for in vivo editing of the Ush2A gene site in mouse eyes. We use the 151nt arRNA (SEQ ID NO: 32) to conduct this experiment. That is, the test arRNA used in this example comprises a counter position targeting base C to the target A; when the arRNA hybridizes with the target RNA, an unmatched A-C (mismatch) can be formed at the target A. At the same time, the 5' and 3' ends of targeting base C of the arRNA are respectively 75nt base sequences that are completely complementary to the target sequence. Since the sequence of the mouse Ush2A gene is different from that of the human USH2A gene, and the test mice do not carry Usher syndrome-related mutation, the 151nt arRNA used in this example is an arRNA targeting the mouse Ush2A-related site, not an arRNA targeting human USH2A gene sequence. We insert the arRNA coding sequence into the AAV vector after the U6 promoter starting site, and package the AAV vector with the inserted arRNA sequence into AAV2 and AAV5 at 1×10¹³GC/mL. Particularly, construction of the AAV vector (see SEQ ID NO: 16 for the constructed vector, wherein the arRNA coding sequence is represented by capital letters, capital or lowercase letter of the same letter does not represent base difference) and packaging of the AAV virus are all finished by Guangzhou PackGene Biotech Co., Ltd..

6-8 weeks aged C57 male mice of SPF grade are used in this example, and there are 8 mice in each of the experimental group (AAV2 and AAV5 groups) and the control group (PBS), totally 24 mice. 2 µL of AAV or PBS is respectively injected into the right eyes of mice in the experimental group and control group through the vitreous. On day 7 (later marked as week 1) and day 14 (later marked as week 2) after injection, 4 mice in each group are sacrificed, taking out their right eyes to mince with scissors into small pieces of 50-100mg. After grinding with liquid nitrogen, the samples are collected with 800µL TRIzol (Invitrogen, 15596018). RNA extraction is performed by using the Direct-zol RNA Miniprep kit (Zymo Resaerch, R2052). 1000ng of the extracted RNA from each sample is reverse transcribed with TransScript^{®} One-Step gDNA Removal and cDNA Synthesis SuperMix Kit (TransGen Biotech, AT311) to synthesize cDNA. 1 µL of the reverse transcription product for each mouse is taken to perform PCR with two primers having the sequences of ggagtgagtacggtgtgcCCCATCTCTTTGGCTTGGAACCAT (SEQ ID NO: 22) and gagttggatgctggatggCTTTTCTCTCTGCTCCACTGTGAAGTT (SEQ ID NO: 24) and Q5 hot-start enzyme (NEB, M0494L). The PCR products are used to build library by using Hi-TOM kit (Novogene, REF PT045), then fulfilling the next-generation sequencing and data analysis (sequencing and data analysis methods are the same as those in Example 2).

The results are shown in Fig. 14. It can be seen from this Figure that in the mouse in vitro test, the editing efficiency of AA5 in mouse eyeball cells is higher than that of AAV2 when injecting through mouse vitrea. However, it can be clearly seen from the Fig. that the editing efficiency of AAV2 or AAV5 is low. For example, on day 7 the average editing efficiency of AAV2 is 0.5%, which is not significantly different from the PBS group. Although the average efficiency of AAV5 is about 1%, the 4 parallel tests are significantly inconsistent in editing efficiency, wherein the editing efficiency of one of them is more than 2%, and that of the other three is about 0.5%. On day 14 the editing efficiency in AAV2 group is also nearly 1%, and the average editing efficiency in AAV5 group is 2%, and the editing efficiency for one of the samples is nearly 5%. Through this test, we find the following problems: firstly, in the eyes of mice, it seems that the editing efficiency of AAV5 is higher than that of AAV2; secondly, the data from different mice is quite different, this may indicate that the conditions of our vitreous injection are not stable, resulting in a big difference in each injection; finally, there is a tendency to rise of the editing efficiency from day 7 to day 14, which means that if the detection time is delayed, higher editing efficiency may be detected.

Therefore, we restart the eye ball test of mice and optimize the test system as follows: 1) since the editing efficiency of AAV5 is higher than that of AAV2, we retain AAV5 in this experiment and test AAV virus of another serum type: AAV8; 2) for the problem of unstable editing effects of vitreous injection, we change the injection condition to subretinal space injection, and after injection, success of the mouse's subretinal space injection is confirmed through Optical Coherence Tomography(OCT); 3) the detection time is modified to day 14 (week 2), day 28 (week 4), day 42 (week 6), and day 56 (week 8); 4) since the Usher syndrome is caused by degenerative lesions of the cone cells and rod cells in retina, this experiment only detects the editing efficiency of Ush2A in retina. Compared with detecting the editing efficiency of the entire eyeball, only detecting the editing efficiency of Ush2A in retina is more instructive for future treatment.

In this experiment, 6-8 weeks aged C57 male mice of SPF -level are used, 20 mice in each testing group (AAV5, AAV8 group), and 12 mice in the control group (NaCl).

The method of subretinal space injection is as follows: 1-2 drops of tropicamide compound eye drop is dropped into the binoculus of animals for mydriasis, and the animals are general anesthetized through muscle injection of Zoletil^{®} 50 (50 mg/kg, 50 mg/ml). The anesthetized mice are made to lie on the side on the operation table. Polyhone iodine cotton balls are used to disinfect the orbital skin of the mice. A micro-tweezer with teeth is used to clamp the conjunctiva on the side of the eyeball, and a micro-scissor is used to cut the fascia under the bulber conjunctiva to expose some sclera. The micro-tweezer is kept still, covering the cornea with a cover glass; Kabham's eye drop is used to eliminate the air, and then a disposable insulin needle is used to form a small angle with the sclera to perform puncture of sclera and choroid directly to the junction of retinal epithelium and retina to form a channel. The micro-tweezer is continuously kept still, then seceding the insulin needle and replacing it with a 33G microscope syringe. The microscope syringe is made to pierce from the channel opening to sub-retina, stopping the needle for at least 3-5s after 1µL of the agent is slowly administered. The microscope syringe is continuously kept in the channel for about 30s under good anesthesia state of the animal (to minimize the volume of leakage when seceding the needle). The needle needs to be cleaned after each mouse is administered to ensure that there is no cross-contamination. After binoculus injection is completed, OCT scan is used to confirm that the injection position is located in subretinal space. The animal injection is determined successful if the subretinal space of both eyes is successfully injected. 5 (test group) or 3 (control group) mice are respectively euthanized on day 14, 28, 42, and 56 after injection. The left eye of the mouse is taken out to cut the cornea 360° along the limbus with a scalpel. The iris and crystalline is removed to take out the retina- choroid -sclera complex, removing the outermost sclera to cut into small pieces and put them in a sterile EP tube with 400µl pre-cold TRIzol (Thermo, 15596018), then mixing with a tissue homogenizer. RNA is extracted by using Direct-Zol RNA Microprep kit (Zymo Resaerch, R2062). 1000ng of the extracted total RNA from each sample is reverse transcribed with reverse transcription kit from TransGen Biotech (TransGen Biotech, AH341-01). 5µL of the reverse transcribed cDNA is used to perform PCR by using two primers with sequences of ggagtgagtacggtgtgcCCCATCTCTTTGGCTTGGAACCAT (SEQ ID NO: 22) and gagttggatgctggatggCTTTTCTCTCTGCTCCACTGTGAAGTT (SEQ ID NO: 24) and Q5 hot-start enzyme (NEB, M0494L). The PCR products are used to build library by using Hi-TOM kit (Novogene, REF PT045), then fulfilling the next-generation sequencing and data analysis (sequencing and data analysis methods are the same as those in example 2).

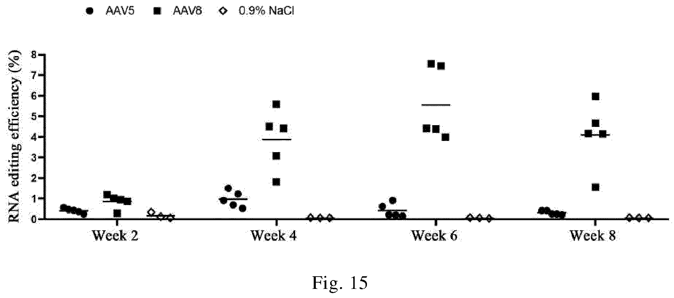

The results are shown in Fig. 15. It can be clearly seen from this Figure that, comparing to the highest average editing efficiency of 2% in the first test (Fig. 14), this experiment can reach an editing efficiency of more than 5%. In addition, since in this experiment the mouse retina is stripped off to collect samples for sequencing instead of cutting and grinding the whole eyeball of the mouse, it can better reflect the actual treatment effect produced during the actual treatment. It can be proved through this experiment that, LEAPER technology can edit Ush2A endogenous gene sites in retina cells in in vitro experiment of mice, and this editing can last at least 2 months in mice.

Based on the results of the above Examples, this application fully proves the feasibility of treatment of the Usher syndrome by using the technical solution disclosed in this application.

### References:

1. Boughman, J. A., Vernon, M., & Shaver, K. A. (1983). Usher syndrome: definition and estimate of prevalence from two high-risk populations. Journal of chronic diseases, 36(8), 595-603.
2. Cox, D. B., Gootenberg, J. S., Abudayyeh, O. O., Franklin, B., Kellner, M. J., Joung, J., & Zhang, F. (2017). RNA editing with CRISPR-Cas13. Science, 358(6366), 1019-1027.
3. Davenport, S. L. H., & Omenn, G. S. (1977). The heterogeneity of Usher syndrome. Vth Int. Conf. Birth Defects, Montreal*.*
4. Grøndahl, J. (1987). Estimation of prognosis and prevalence of retinitis pigmentosa and Usher syndrome in Norway. Clinical genetics, 31(4), 255-264.
5. Liu, X., Bulgakov, O. V., Darrow, K. N., Pawlyk, B., Adamian, M., Liberman, M. C., & Li, T. (2007). Usherin is required for maintenance of retinal photoreceptors and normal development of cochlear hair cells. Proceedings of the National Academy of Sciences, 104(11), 4413-4418.
6. Merkle, T., Merz, S., Reautschnig, P., Blaha, A., Li, Q., Vogel, P., ... &Stafforst, T. (2019). Precise RNA editing by recruiting endogenous ADARs with antisense oligonucleotides. Nature biotechnology, 37(2), 133.
7. Neuhaus, C., Eisenberger, T., Decker, C., Nagl, S., Blank, C., Pfister, M., ... & Beck, B. (2017). Next-generation sequencing reveals the mutational landscape of clinically diagnosed Usher syndrome: copy number variations, phenocopies, a predominant target for translational read-through, and PEX 26 mutated in Heimler syndrome. Molecular genetics & genomic medicine, 5(5), 531-552.
8. Otterstedde, C. R., Spandau, U., Blankenagel, A., Kimberling, W. J., &Reisser, C. (2001). A new clinical classification for Usher's syndrome based on a new subtype of Usher's syndrome type I. The Laryngoscope, 111(1), 84-86.
9. Pollard, K. M., Cauvi, D. M., Toomey, C. B., Morris, K. V., &Kono, D. H. (2013). Interferon-y and systemic autoimmunity. Discovery medicine, 16(87), 123.
10. Qu, L., Yi, Z., Zhu, S., Wang, C., Cao, Z., Zhou, Z., ... & Bao, Y. (2019). Programmable RNA editing by recruiting endogenous ADAR using engineered RNAs. Nature biotechnology, 37(9), 1059-1069.
11. Xu, L., Wang, J., Liu, Y., Xie, L., Su, B., Mou, D., ... & Zhao, L. (2019). CRISPR-edited stem cells in a patient with HIV and acute lymphocytic leukemia. New England Journal of Medicine, 381(13), 1240-1247.
12. Usher, C. H. (1914). On the inheritance of retinitis pigmentosa; with note of cases. Royal London Opthalmol Hosp Rep, 19, 130-236.
13. https://en.wikipedia.org/wiki/Nyctalopia

## Claims

1. A method for targeted editing of target RNA in a cell based on LEAPER technology, wherein the target RNA is an RNA containing a G to A mutation in a USH2A gene transcript, comprising:
introducing a construct comprising an adenosine deaminase recruiting RNA (arRNA) for editing the target RNA or a construct encoding the arRNA into the cell, wherein the arRNA comprises a complementary RNA sequence that hybridizes to the target RNA, and wherein the arRNA is capable of recruiting adenosine deaminase acting on RNA (ADAR), thereby the target adenosine in the target RNA is deaminated.

2. The method according to claim 1, wherein the target RNA is a pre-mRNA.

3. The method according to claim 1 or 2, wherein the length of the arRNA is about 151-61 nt, 131-66 nt, 121-66 nt, 111-66 nt, 91-66 nt, or 81-66 nt.

4. The method according to claim 3, wherein the length of the arRNA is any natural number selected from 66nt-131nt.

5. The method according to any one of claims 1-4, wherein the length from the targeting base to the 3' end in the arRNA is ≥7nt, ≥10nt, ≥15nt, preferably 16-55nt, 20-50nt, 25-45nt, 25-35nt, or 25-30nt.

6. The method according to any one of claims 1-5, wherein the length from the targeting base to the 5' end in the arRNA is ≥25nt, ≥30, ≥35, ≥45, preferably 46-90nt, 50-85nt, 55-80nt, 60-75nt, or 65-70nt.

7. The method according to any one of claims 1-6, wherein a targeting base is introduced into the arRNA to pair with the target A in the target sequence, and the preference order of the targeting base from high to low is C, A, U, or G.

8. The method according to any one of claims 1-7, wherein the target RNA is an RNA comprising a transcribed human USH2A gene NM_206933.2(USH2A)_c.11864 G>A (p.Trp3955Ter) mutation site.

9. The method according to any one of claims 1-8, wherein the arRNA is selected from the group consisting of: SEQ ID NO: 23, SEQ ID NO: 25, SEQ ID NO: 26, SEQ ID NO: 27, SEQ ID NO: 28, SEQ ID NO: 29, SEQ ID NO: 30, SEQ ID NO: 17, SEQ ID NO: 18, SEQ ID NO: 19, SEQ ID NO: 11, SEQ ID NO: 12, SEQ ID NO: 6, SEQ ID NO: 7, SEQ ID NO: 8, SEQ ID NO: 9, SEQ ID NO: 2, SEQ ID NO: 3, and SEQ ID NO: 4.

10. The method according to any one of claims 1-9, wherein the arRNA is selected from the group consisting of: SEQ ID NO: 7, SEQ ID NO: 8, and SEQ ID NO: 9.

11. The method according to any one of claims 1-10, wherein the construct is a linear nucleic acid, a viral vector, or a plasmid.

12. The method according to claim 11, wherein the viral vector is an adeno-associated virus (AAV) vector or a lentiviral vector.

13. The method according to claim 12, wherein the AAV vector is introduced into the cell by infection after being packaged into an AAV2, AAV5 or AAV8 capsid.

14. The method according to claim 13, wherein the AAV vector is introduced into the cell by infection after being packaged into an AAV8 capsid.

15. The method according to any one of claims 1-14, wherein the cell is an optic nerve cell or auditory nerve cell of a mammalian.

16. An arRNA for targeted editing of the target RNA containing a G to A mutation in a USH2A gene transcript, comprising or consisting of a sequence selected from the group consisting of: SEQ ID NO: 23, SEQ ID NO: 25, SEQ ID NO: 26, SEQ ID NO: 27, SEQ ID NO: 28, SEQ ID NO: 29, SEQ ID NO: 30, SEQ ID NO: 17, SEQ ID NO: 18, SEQ ID NO: 19, SEQ ID NO: 11, SEQ ID NO: 12, SEQ ID NO: 6, SEQ ID NO: 7, SEQ ID NO: 8, SEQ ID NO: 9, SEQ ID NO: 2, SEQ ID NO: 3, and SEQ ID NO: 4.

17. A construct or delivery vector comprising the arRNA according to claim 16.

18. The construct or delivery vector according to claim 17, which is a plasmid, virus, liposome, or lipid nanoparticle.

19. The construct or delivery vector according to claim 18, which is an adeno-associated virus (AAV) or a lentivirus.

20. The construct or delivery vector according to claim 19, wherein the virus is AAV2, AAV5, or AAV8.

21. A cell obtained by the editing method according to any one of claims 1-15.

22. A method for treating Usher type II syndrome in an individual, comprising correcting a G to A mutation associated with Usher type II syndrome in a cell of the individual by the method according to any one of claims 1-15.

23. The method according to claim 22, comprising introducing the arRNA according to claim 16 or the construct or delivery vector according to any one of claims 17-20 into a subject.

24. The method according to claim 23, wherein the arRNA according to claim 16 or the construct or delivery vector according to any one of claims 17-20 is introduced into the subretinal space of the subject.

25. Use of the arRNA according to claim 16 or the construct or delivery vector according to any one of claims 17-20 in the preparation of a medicament for treating Usher type II syndrome.
